# EUROPÄISCHE PATENTSCHRIFT

(11) **EP 2 422 843 B1**
(45) Veröffentlichungstag und Bekanntmachung des Hinweises auf die Patenterteilung: **04.03.2015**
(21) Anmeldenummer: 11176439.5
(22) Anmeldetag: 03.08.2011
(51) Int. Cl.: A61N 1/362

(54) **Implantierbares elektronisches Therapiegerät**
Implantable electronic therapy device
Appareil de thérapie électronique implantable

(30) Priorität: 30.08.2010 US 377982 P
(43) Veröffentlichungstag der Anmeldung: 29.02.2012
(73) Patentinhaber: BIOTRONIK SE & Co. KG, 12359 Berlin (DE)
(72) Erfinder: Dörr, Thomas, 12437 Berlin (DE)
(74) Vertreter: Lindner-Vogt, Karin L.

(56) Entgegenhaltungen:
- EP-A2- 0 310 026
- EP-A2- 1 142 608
- WO-A2-01/30436
- FR-A1- 2 861 996
- US-A1- 2003 100 925
- US-A1- 2004 002 741
- US-A1- 2006 224 201
- US-B1- 6 993 385
- US-B1- 7 027 863

## Beschreibung

Die Erfindung betrifft ein implantierbares elektronisches Therapiegerät mit einer Therapieeinheit, einer Herzratenerfassungseinheit, und einer Auswerte- und Steuereinheit, von denen die Therapieeinheit ausgebildet ist eine antitachykarde Therapie zu liefern, die Herzratenerfassungseinheit dazu ausgebildet ist, aus einem Eingangssignal eine ventrikuläre Herzrate zu bestimmen und die Auswerte- und Steuereinheit mit der Therapieeinheit und der Herzratenerfassungseinheit verbunden und ausgebildet ist, die Therapieeinheit zur Abgabe einer antitachykarden Therapie anzusteuern, wenn die Herzratenerfassungseinheit einen Anstieg der Herzrate über einen vorbestimmten Schwellwertes erfasst.

Derartige implantierbare elektronische Therapiegeräte sind beispielsweise als implantierbare Cardioverter / Defibrillatoren (ICDs) bekannt, die eine antitachykarde Therapie wie beispielsweise einen Kardioversionsschock oder einen Defibrillationsschock, dann auslösen, wenn deren Auswerte- und Steuereinheit vorgegeben Kriterien erfasst, die eine ventrikuläre Tachykardie (VT) oder eine supraventrikuläre Tachykardie (SVT) anzeigen, insbesondere einen Anstieg der ventrikulären Herzrate über einen vorgegebenen Schwellwert. Hierzu werden Herzraten typischerweise in verschieden Frequenzbereichen, sogenannten VT-Zonen, zugeordnet.

Aus dem Stand bekannte antitachykarde Stimulatoren, insbesondere ICDs verwenden zur Steuerung der antitachykarden Therapie in den VT-Zonen verschiedene rhythmologische Zähler und EKG-Morphologiekriterien, um eine Therapieentscheidung abzuleiten. Ebenso kommen Algorithmen zum Einsatz, die Kombinationen solcher Rhythmus- und Morphologieparameter betrachten.

Die US20030100925 offenbart eine Methode zur Bestimmung der Kontraktilität und die Nutzung der Daten der Kontraktilität des Herzens zur Bestimmung der mechanischen Eigenschaften des Herzens.

Die EP1142608 offenbart die Behandlung von vagovasalen Synkopen durch eine Erhöhung der Herzrate.

Die EP0310026 ist auf die Auswertung der Daten einer Elektrode mit nur einem Sensor gerichtet, insbesondere auf die impedanzbasierte Auswertung der Pulmonalen- und der Kardialenaktivität aus einem IEGM.

Aus US 6,873,870 ist es darüber hinaus bekannt, allgemeine hämodynamische Sensoren, wie Blutdruck und Fluss in die Bewertung der Tachyarrhythmie nach hämodynamischer Relevanz einzubeziehen.

Der Erfindung liegt die Aufgabe zugrunde, eine antitachykarde Therapie im Falle einer VT oder SVT immer erst dann abzugeben, wenn sich eine hämodynamische Relevanz der Tachykardie abzeichnet. Ziel der Erfindung ist insbesondere dass unabhängig vom Ursprung einer schnellen ventrikulären Rate eine antitachykarde Therapie, insbesondere eine Schocktherapie, immer nur dann abgegeben wird, wenn die ventrikuläre Rate zu einer hämodynamischen Einschränkung führt.

Erfindungsgemäß wird diese Aufgabe durch ein implantierbares elektronisches Therapiegerät mit einer Therapieeinheit, einer Herzratenerfassungseinheit, einer Kontraktilitätsbestimmungseinheit und einer Auswerte- und Steuereinheit gelöst von denen:
- die Therapieeinheit ausgebildet ist eine antitachykarde Therapie zu liefern,
- die Herzratenerfassungseinheit dazu ausgebildet ist, aus einem Eingangssignal eine ventrikuläre Herzrate zu bestimmen
- die Kontraktilitätsbestimmungseinheit dazu ausgebildet ist, aus einem Eingangssignal ein Kontraktionssignal zu generieren, dass eine Kontraktilität einer Herzkammer widerspiegelt und
- die Auswerte- und Steuereinheit mit der Therapieeinheit, der Herzratenerfassungseinheit und der Kontraktilitätsbestimmungseinheit verbunden und ausgebildet ist, die Therapieeinheit zur Abgabe einer antitachykarden Therapie anzusteuern, wenn
   a) die Herzratenerfassungseinheit einen Anstieg der Herzrate über einen vorbestimmten Schwellwertes erfasst
      und
   b) die Kontraktilitätsbestimmungseinheit ein Kontraktionssignal liefert, dass dem Anstieg der Herzrate nicht physiologisch adäquat ist.

Oder mit anderen Worten: erfindungsgemäß besitzt ein dauerimplantierbares elektronisches Therapiegerät zur antitachykarden Therapie des Herzens, mindestens
- eine Vorrichtung zur Erfassung einer dem Kontraktionszustand bzw. Kontraktionsdynamik entsprechenden Größe (bevorzugt: unipolare intrakardiale Impedanz) und
- eine Einheit zur Erfassung der ventrikulären Herzrate und
- eine Auswerte- und Therapiesteuereinheit, wobei die Auswerte- und Therapiesteuereinheit eine antitachykarde Therapie immer dann einleitet, wenn die ventrikuläre Rate einer VT entspricht und der erfasste Kontraktilitätsverlauf bezogen auf den Frequenzanstieg nicht einem physiologisch zu erwartendem Verlauf entspricht.

Im Unterschied zu bekannten implantierbaren elektronischen Therapiegeräten ist das erfindungsgemäße Gerät somit ausgebildet eine antitachykarde Therapie, insbesondere eine Schocktherapie, immer nur dann abzugeben, wenn die Tachykardie tatsächlich zu einer hämodynamischen Einschränkung führen kann, indem neben der Herzrate auch die Kontraktilität erfasst und in die Therapieentscheidung mit einbezogen wird. Die Therapieentscheidung ist dabei unabhängig vom Ursprung der gesteigerten ventrikulären Rate. Auf diese Weise wird eine Reduktion inadäquater Schocks oder anderer Therapien und eine Steigerung der Therapieeffizienz von ICD-System o. ä. möglich. Das erfindungsgemäße Gerät benötigt nur die ohnehin vorhandene rechtsventrikuläre oder eine vergleichbare Elektrode, d.h. es bestehen keinerlei zusätzliche Anforderungen an Elektroden oder Sensoren.

Die Erfindung schließt die Beobachtung ein, dass es ein Nachteil aller bekannten Lösungen ist, dass zur Therapieentscheidung entweder nur der Ursprungs einer Tachykardie oder die Dauer der Tachykardie ausgewertet werden. Dies führt dazu, dass häufig VTs zu früh mit einem Schock therapiert werden, ohne die Chance einer Spontanterminierung abzuwarten oder aber hämodynamisch relevante SVTs (z.B. AV-Reentry) nicht behandelt werden, obwohl hier eine Schocktherapie eine angemessene Therapieoption trotz supraventrikulärem Arrhythmieursprungs darstellt.

Die in US 6,873,870 beschriebenen Sensoren zur hämodynamischen Klassifikation der Tachyarrhythmie in hämodynamisch relevante und nicht relevante Tachyarrhythmien haben den Nachteil, dass zum einen zusätzliche Sensoren für Druck und/oder Fluss benötigt werden. Außerdem geht US 6,873,870 nicht auf den physiologische Zusammenhang zwischen Herzfrequenz und Kontraktilität ein und ignoriert damit einen sehr frühzeitigen Indikator einer sich andeutenden hämodynamische Relevanz, da langsame VTs über klassische hämodynamische Parameter wie Druck und Fluss aufgrund vielfältiger Kompensationsmechanismen zunächst nicht diagnostiziert werden können. Die reduzierte oder unveränderte Kontraktilität als eine der Regelgrößen für Herzzeitvolumen hingegen kann sofort als Diskriminator genutzt werden.

Die Erfindung macht sich das physiologische Prinzip zunutze, dass jede physiologische Steigerung des Herzzeitvolumens durch eine Steigerung der Herzfrequenz mit einer gleichzeitigen Steigerung der Kontraktionskraft und Kontraktionsgeschwindigkeit - also der Kontraktilität - einhergeht. Dem gegenüber wird bei einer nichtphysiologischen bzw. pathophysiologische Herzfrequenzsteigerung die Kontraktionskraft und Kontraktionsgeschwindigkeit nicht gesteigert oder als kompensatorische Maßnahme sogar reduziert. Erfindungsgemäß wird daher die Therapieentscheidung von der Prüfung des physiologischen Zusammenhangs zwischen Herzfrequenzsteigerung und einen dem Kontraktionsverlauf entsprechenden Parameter abhängig gemacht, d.h. eine Plausibilitätsprüfung für den Frequenzanstieg durchgeführt.

Entscheidender Vorteil dieser Methode gegenüber den schon bekannten "echten hämodynamischen" Messungen ist, dass sie nur eine relativ einfache Messung, beispielsweise eine unipolare Impedanzmessung zur Ermittlung eines dem Kontraktionszustand entsprechenden "relativen" Parameters erfordert. Das eine solche Messung für die Implantatsteuerung hinreichend ist, ist mit dem CLS-Prinzip (CLS: Closed Loop Stimulation) hinreichend nachgewiesen. Hier wird mittels einer unipolaren Impedanzmessung die fehlende Chronotropie durch die Information des Kontraktionszustandes ersetzt und die Ratenadaption des Schrittmachers gesteuert. Wichtig ist dabei, dass diese unipolare Impedanzmessung keine wirkliche hämodynamische Größe erfasst, wie sie z.B. in US 6,873,870 vorausgesetzt wird. Dies ergibt sich auch aus den aktuellen Bestrebungen, hämodynamische Kenngrößen mittels multipolarer Impedanzmessungen zu erfassen, da die unipolare Messung nicht hinreichend ist.

Vorteilhafte Ausgestaltungen des erfindungsgemäßen implantierbaren elektronischen Therapiegeräts sind die folgenden:
Das erfindungsgemäße implantierbare elektronische Therapiegerät ist vorzugsweise ein implantierbarer Cardioverter/Defibrillator (ICD), ein biventrikulärer Herzstimulator für die Resynchronisationstherapie (CRT-D) und/oder ein antitachykarder Herzschrittmacher.

Alternativ oder zusätzlich kann das implantierbare elektronische Therapiegerät eine Medikamentenpumpe aufweisen.

Auch kann das implantierbare elektronische Therapiegerät ein Stimulator zur Modulation der kardialen Kontraktilität (siehe z.B.: Impulse Dynamics CCS device) sein.

Das implantierbare elektronische Therapiegerät kann auch ein "Leadless-ICD" sein.

Das implantierbare elektronische Therapiegerät ist vorzugsweise dazu ausgebildet, die Wahrnehmung der Kontraktilitätsinformation mittels einer kontinuierlichen intrakardialen Impedanzmessung an einer ventrikulären Elektrode - entsprechend dem CLS-Prinzip - durchzuführen. Dies kann wahlweise im rechten Ventrikel, im linken Ventrikel, bipolar oder unipolar erfolgen. Das physiologische Grundprinzip der CLS-Schrittmacherfrequenzanpassung beruht darauf, dass bei einem physiologischem Herzfrequenzanstieg (Chronotropie) immer auch die Kontraktilität (Inotropie) des Herzens zunimmt. Liegt bei einem Patienten eine Sinusknotenerkrankung und damit eine chronotrope Inkompetenz vor, wird im physiologischen Belastungsfall zwar die Herzfrequenz nicht mehr adäquat gesteigert, jedoch immer noch die Kontraktilität erhöht. Wenn eine Messung der Kontraktilität möglich ist, so kann bei diesen Patienten dann auch die Frequenzanpassung durch den Herzschrittmacher entsprechend gesteuert werden. Hierzu wird nun unmittelbar nach jeder ventrikulären Erregung eine kontinuierliche unipolare Impedanzkurve an der ventrikulären Elektrodenspitze aufgezeichnet. Dieses Impedanzsignal wird durch die unterschiedliche Leitfähigkeit des umgebenden Herzmuskelgewebes (geringere Leitfähigkeit) und des Blutes (höhere Leitfähigkeit) geprägt. Bei Kontraktion des Ventrikels verringert sich die Leitfähigkeit im Bereich der Elektrodenspitze, das zunehmend Blutvolumen durch die Kontraktion verdrängt wird. Um eine physiologische Steuergröße ableiten zu können, werden die Impedanzkurven mit jeweils unter nachgewiesenen Ruhebedingungen aufgezeichneten Referenzimpedanzkurven verglichen und eine Differenzfläche zwischen aktueller und Referenzimpedanzkurve bestimmt. Die Flächendifferenz ist dann das Maß für die relative gestiegene Kontraktionsdynamik für diesen einen Herzzyklus und wird dann als Maß für die Ratenanpassung des CLS-Schrittmachers genutzt. Vorteil dieser Methode ist das sehr robuste und relativ einfache Messprinzip der unipolaren Impedanzmessung mit einen guten Signal-Rausch-Verhältnis, so dass hier Schlag-zu-Schlag-Messungen ohne nötige Mittelwertbildungen möglich sind. Allerdings ist die Flächendifferenz der Impedanzkurven nur eine relative Kenngröße der Kontraktionsdynamik und kann weder in einen Absolutwert der Kontraktilität oder Schlagvolumen umgerechnet werden, noch im Langzeitverlauf ausgewertet werden. D.h. das Messsignal ist nicht langzeitstabil, daher müssen die Referenzkurven kontinuierlich angepasst/aktualisiert werden.

Die Erfindung macht sich nun den selben physiologischen Ansatz zu Nutze, um mittels einer Plausibilitätsprüfung bei den zumeist chronotrop kompetenten ICD-Patienten die Herzfrequenzsteigerungen zu prüfen, ob diese Folge einer physiologische Frequenzanpassung sind oder aber als unphysiologisch eingestuft werden, wenn die zu erwartende Kontraktionssteigerung bei Frequenzsteigerung ausbleibt oder einen unerwarteten Verlauf nimmt. Auch dazu ist das im CLS-Prinzip genutzte Messverfahren hinreichend.

Gemäß einer alternativ bevorzugten Ausführungsvariante ist das implantierbare elektronische Therapiegerät dazu ausgebildet, die Wahrnehmung der Kontraktilitätsinformation mittels transkardialer Impedanzmessung gemäß dem HDS-Prinzip durchzuführen. Hier wird mittels einer 4-polaren Impedanzmessung ein Korrelat für die linksventrikuläre Volumenänderung während einer Ventrikelkontraktion bestimmt, indem kontinuierlich Messstrompulse an z.B. der rechts- oder linksventrikulären Elektrode bipolar eingespeist werden und an der entsprechenden anderen Elektrode die durch die Messströme hervorgerufenen zusätzlichen Spannungen gemessen werden. Auch hier wird die Leitfähigkeitsänderung durch das unterschiedliche Blutvolumen im linken Ventrikel genutzt, um eine Kenngröße der Kontraktionsdynamik zu erhalten. Vorteil gegenüber der CLS-Messung ist die Möglichkeit, den linksventrikulären Kontraktionsverlauf abbilden zu können und damit insbesondere die Dekompensation des linken Vetrikels abzubilden. Allerdings ist diese Messung auf eine bipolare linksventrikuläre Elektrode angewiesen und besitzt gegenüber der CLS-Messung ein schlechteres Signal-Rausch-Verhältnis.

Für die erfindungsgemäße Überprüfung des Zusammenhanges zwischen Herzfrequenzsteigerung und Kontraktionsdynamik ist dieses Signal ebenso geeignet.

Gemäß einer weiteren alternativ bevorzugten Ausführungsvariante ist das implantierbare elektronische Therapiegerät dazu ausgebildet, die Wahrnehmung der Kontraktilitätsinformation mittels transthorakaler Impedanzelektrokardiographie durchzuführen. Bei diesem hinlänglich bekannten Verfahren wird die Thoraximpedanzänderung während des Herzschlages ausgewertet. Auch in diesem Signal ist eine Information über die Kontraktionsdynamik zum Zeitpunkt der ventrikulären Kontraktion enthalten (dZ/dt), dass mit dem Verlauf des Herzfrequenzanstiegs verglichen und klassifiziert werden kann. Dieses Methode kann bei Implantaten verwendet werden, die bauartbedingt nicht in der Lage sind, eine lokale Impedanzmessung an der oder den ventrikulären Sonden durchzuführen, d.h. z.B. bei Stimulatoren, die über keine ventrikuläre Elektrode verfügen (z.B. S-ICD) o.ä..

Gemäß noch einer weiteren alternativ bevorzugten Ausführungsvariante ist das implantierbare elektronische Therapiegerät dazu ausgebildet, die Wahrnehmung der Kontraktilitätsinformation mittels intrakardialer Druckmessung durchzuführen. Verfügt das Implantat über die Möglichkeit einer intrakardialen, Pulmonararterien- oder aortalen Druckmessung, so können die Kontraktionsparameter auch aus diesen hämodynamischen Messwerten abgeleitet und erfindungsgemäß mit der Herzfrequenzsteigerung korreliert werden. Allerdings dient dieser Ansatz nur der vollständigen Darstellung der Methode. Bei Verfügbarkeit eines Drucksensors kann zumeist die hämodynamische Relevanz einer Arrhythmie direkt abgeleitet werden.

Vorzugsweise repräsentiert Kontraktilitätssignal die Kontraktionsgeschwindigkeit.

Vorzugsweise ist die Auswerte- und Steuereinheit ausgebildet, eine antitachykarde Therapie wird immer dann einzuleiten, wenn die ventrikuläre Rate einer VT entspricht und der erfasste Frequenzanstieg nicht mit einer positiven Inotropie verbunden ist bzw. verbunden bleibt, d.h. die Kontraktilität nicht zeitgleich mit der Herzrate ansteigt. Oder anders ausgedrückt: die Auswerte- und Steuereinheit ist vorzugsweise ausgebildet, eine Abgabe einer antitachykarden Therapie durch die Therapieeinheit zu unterbinden, wenn die Herzratenerfassungseinheit einen Anstieg der Herzrate über einen vorbestimmten Schwellwertes erfasst und die Kontraktilitätsbestimmungseinheit ein Kontraktionssignal liefert, das anzeigt, dass die entsprechende Kontraktilität der Herzkammer gleichzeitig mit der Herzrate ansteigt

Vorzugsweise wertet die Auswerte- und Steuereinheit das genannte Kriterium wird immer in Kombination mit mindestens einem weiteren rhythmologischen oder IEGM-morphologischem Kriterium aus.

Die Auswerte- und Steuereinheit kann auch ausgebildet sein, einen Anstieg der Herzrate (Frequenzanstieg) immer dann als physiologisch, d.h. nicht therapiepflichtig zu bewerten, wenn ein CLS-Signal ebenfalls einen Frequenzanstieg innerhalb einer entsprechenden Toleranz vorgibt und in diesem Fall eine Abgabe einer antitachykarden Therapie durch die Therapieeinheit zu unterbinden. Bleibt hingegen das CLS-Signal hinter dem beobachteten Frequenzanstieg deutlich zurück, löst die Auswerte- und Steuereinheit eine Abgabe einer antitachykarden Therapie durch die Therapieeinheit aus. Das CLS-Signal wird in an sich bekannter Weise aus einem Vergleich des intrakardialen Impedanzverlaufs bei Ruhe eines Patienten mit einem jeweils aktuellen intrakardialen Impedanzverlauf abgeleitet und spiegelt den jeweiligen hämodynamischen Bedarf eines Patenten wieder. Ein höherer hämodynamischer Bedarf indiziert eine höhere Herzrate.

Der Erfindung soll nun anhand von Ausführungsbeispielen mit Bezug auf die Figuren näher erläutert werden. Von den Figuren zeigt:
- Figur 1:: ein Einkammer-ICD-System;
- Figur 2A - C:: Darstellungen des Kontraktilitätsindex für verschiedene Herzzustände;
- Figur 3:: Beispiele für eine VT/SVT-Diskriminierung:
- Figur 4:: ein vereinfachtes Blockschaltbild eines ICD wie aus Fig. 1; und
- Figur 5:: für eine Impedanzmessung geeignete Stromimpulse.

Bei physiologischem Frequenzanstieg durch eine physische oder psychische Belastung wird neben der Herzfrequenz immer auch die Kontraktilität gesteigert (positive Inotropie).

Erfasst man diese Kenngröße oder eine dieser Kenngröße entsprechenden Hilfsgröße, kann man mittels einer einfachen Plausibilitätsprüfung entscheiden, ob eine physiologische Steigerung der ventrikulären Herzrate vorliegt oder eine Steigerung der Herzfrequenz aufgrund einer pathologischen Störung der Erregungsbildung und -leitung erfolgt ist.

Relativ langsame ventrikuläre Tachykardien (VTs) können in vielen Fällen für einen bestimmten Zeitraum hämodynamisch toleriert werden und sind in dieser Zeit noch nicht therapiepflichtig. Viele dieser langsameren VTs enden durch eine Spontanterminierung. Sinkt während einer solchen VT die Kontraktilität jedoch ab, ist von einer akuten Dekompensation auszugehen und eine antitachykarde Therapie einzuleiten.

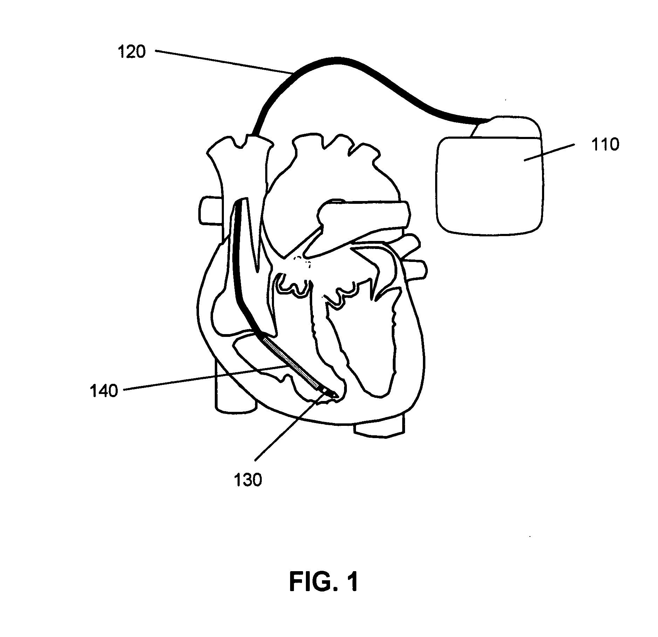

In Figur 1 ist als Implementierungsbeispiel ein Einkammer-ICD-System dargestellt. Ein Impulsgenerator 110 des ICD ist mit einer flexiblen, implantierbaren Elektrodenleitung 120 verbunden. Diese weist an ihrem distalen Ende einen bipolare Wahrnehmungs- und Stimulationspol 130 auf. Zur Defibrillationsschockabgabe sind an der Elektrodenleitung 120 eine distale 140 und optional auch eine proximale Schockwendel angebracht.

Die für die erfindungsgemäße Bestimmung von Kontraktilitätsinformation - und damit des Kontraktionssignals - benötigten Impedanzelektrogramme werden durch Einspeisung von Konstantstromimpulsen über den bipolaren Elektrodenpol 130 und der zur Einspeisung erforderlichen Spannung erfasst.

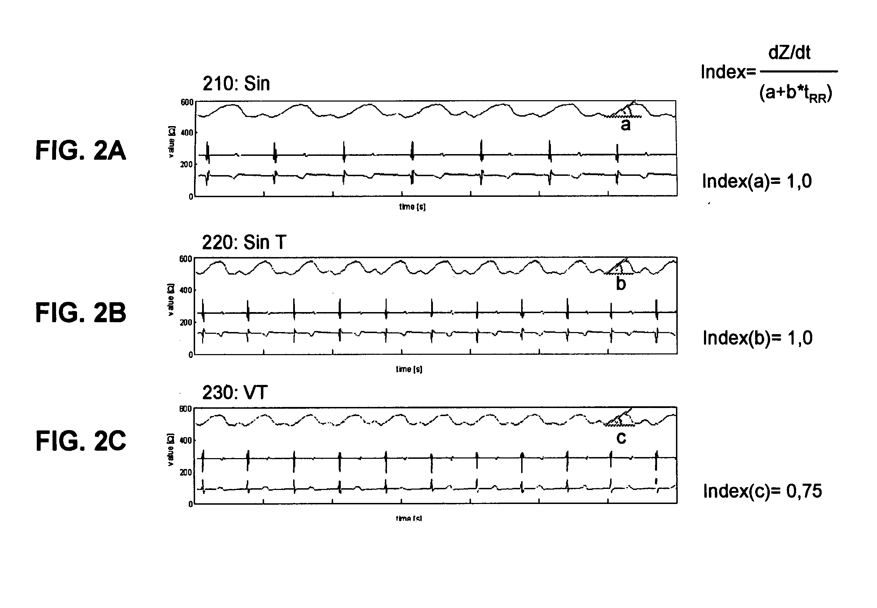

In Figur 2 sind aufgezeichnete typische Impedanzkardiogramme bei unterschiedlichen Rhythmussituationen dargestellt. Im der ersten Aufzeichnung 210 ist ein normfrequenter Sinusrhythmus dargestellt (obere Kurve: Impedanzkardiogramm, Mitte: RV-IEGM, Unten: LV-IEGM). Die Steigung des Impedanzkardiogramms (a) repräsentiert dabei die Kontraktionsgeschwindigkeit und ist damit ein Maß für die Kontraktilität. Aus diesem Wert (dZ/dt) wird Frequenzgewichtet ein entsprechender Kontraktilitätsindex gebildet ("Index"), der eine normierte Aussage erlaubt, ob die Kontraktilität einer entsprechenden physiologischen Herzfrequenz entspricht. Der Kontraktilitätsindex ist hier auf den Wert 1,0 normiert.

In der zweiten Aufzeichnung 220 ist eine belastungsinduzierte Frequenzsteigerung (Dobutamin-induziert) dargestellt. Hier steigt dZ/dt (b) entsprechend an, so dass der berechnete Kontraktilitätsindex ebenfalls etwa 1,0 beträgt und damit einen regulären Kontraktilitätsanstieg mit der Frequenz zeigt.

In der dritten Aufzeichnung 230 ist eine ventrikuläre Tachykardie aufgezeichnet. Hier erreicht die Kontraktionsdynamik dZ/dt (c) einen geringeren Wert als im Falle der Sinustachykardie und zeigt damit einen kleineren frequenznormierten Kontraktilitätsindex von 0,75. Damit ist hier eine Abgrenzung zwischen Sinustachykardie und VT möglich.

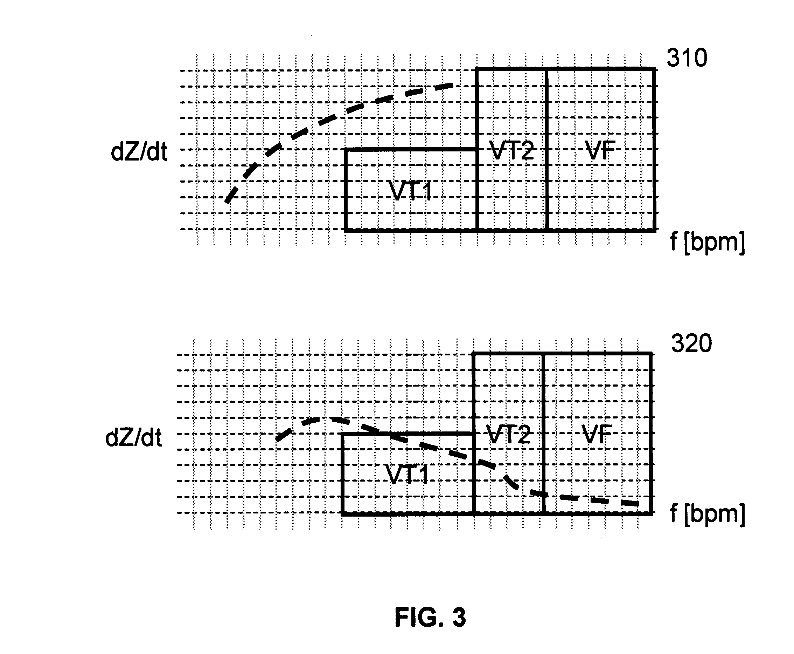

In Figur 3 ist eine alternative Methode zur kontraktilitätsbasierten VT/SVT-Diskriminierung dargestellt. In der oberen Darstellung 310 ist der herzfrequenzabhängige Kontraktilitätsverlauf (ausgedrückt als dZ/dt) für eine belastungsinduzierte Sinustachykardie dargestellt. Die Kurve verläuft oberhalb der als VT gekennzeichneten therapiepflichtigen Zone (VT1). Ab einer kritischen d.h. für Sinustachykardien nicht plausiblen Frequenz wird die Therapiepflicht auf das gesamte Kontraktilitätsspektrum ausgeweitet (VT2/VF). In der unteren Darstellung 320 ist der für eine VT zu erwartende Kontraktilitätsverlauf eingezeichnet. Zu Beginn der VT ist diese unter Umständen noch in einem nicht therapiepflichtigen Bereich, d.h. außerhalb von VT1. Mit zunehmender Frequenz oder Dauer der VT sinkt die Kontraktilität jedoch in den Therapiebereich ab.

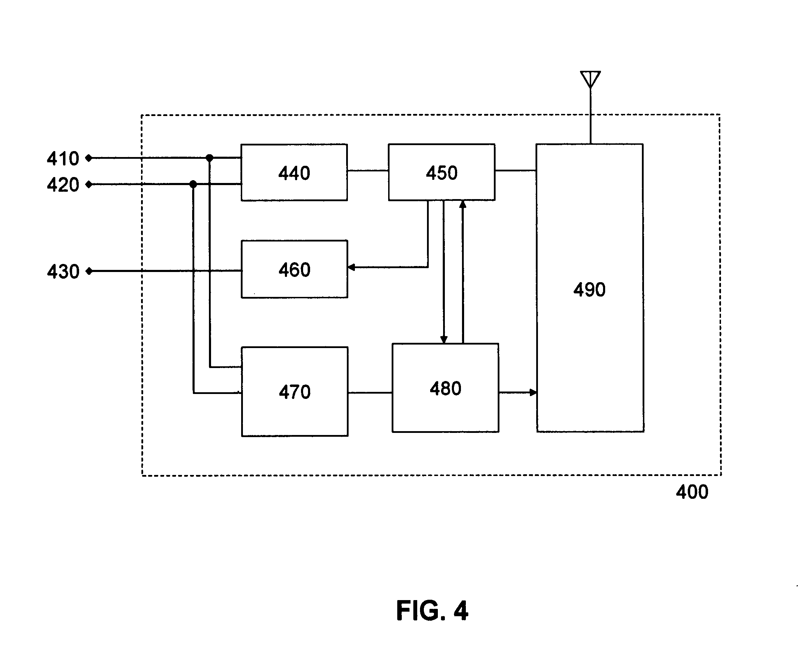

Figur 4 zeigt das Blockschaltbild des erfindungsgemäß erweiterten ICD 400 aus Abbildung 1. Dieser ist mit einer bipolaren Stimulations- und Wahrnehmungselektrode 410, 420 und einer Schockwendel 430 verbunden.

Ein an der bipolaren Wahrnehmungselektrode 420, 430 anliegendes intrakardiales Elektrokardiogramm (IEGM) wird zunächst in einer konventionellen Sensingstufe 440 wahrgenommen und in einer anschließenden Rhythmusbewertungseinheit 450 klassifiziert. Wird eine Tachyarrhythmie festgestellt, so initiiert die Rhythmusbewertungseinheit 450 normalerweise sofort einer Therapieschockabgabe via Therapieeinheit 460.

Erfindungsgemäß ist der ICD jedoch um eine impedanzbasierte Kontraktilitätsbestimmungseinheit 470 und eine zusätzliche Steuereinheit 480 erweitert. Mindestens dann, wenn die Rhythmusbewertungseinheit 450 schnelle Intervalle (z.B. 2 konsekutive Intervalle innerhalb einer VT-Zone) wahrnimmt, wird die zusätzliche Steuereinheit 480 informiert. Diese Steuereinheit aktiviert dann die Kontraktilitätsbestimmungseinheit 470 und bestimmt den ventrikulären Kontraktilitätsindex mittels Impedanzkardiographie an der bipolaren RV-Elektrode. Das Ergebnis dieser Überwachung wird von der zusätzlichen Steuereinheit 480 ausgewertet. Entspricht der Kontraktilitätsindex einer physiologischen Frequenzsteigerung oder zeigt diesem im Frequenzbereich einer VT einen hämodynamisch akzeptablen Wert, so wird die antitachykarde Therapie insgesamt oder mindestens eine Schocktherapie inhibiert. Zeigt der Index einen frequenzgewichteten Kontraktilitätseinbruch, so wird die antitachykarde Therapie freigegeben bzw. eine Schocktherapie forciert.

Die Daten der Rhythmusklassifikation und der ermittelte Kontraktilitätsindex werden in einer Einheit 490 zur Speicherung von Diagnostikdaten aufgezeichnet und dem Anwender optional telemedizinisch zur Verfügung gestellt.

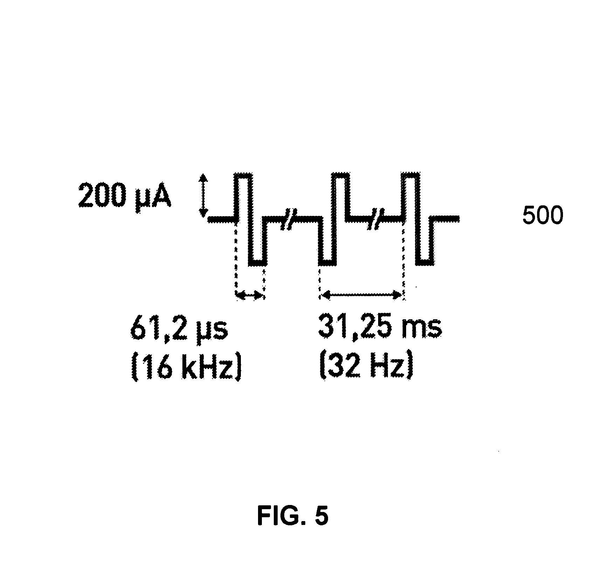

In der Figur 5 sind typische Stromimpulse 500 zur kontinuierlichen intrakardialen Impedanzmessung dargestellt. Diese werden kontinuierlich, jeweils zwischen dem bipolaren Pol der rechtsventrikulären Elektrode abgegeben. Die Aufzeichnung der benötigten Spannung für die Konstantstromeinspeisung ergibt dann das Impedanz-Elektrokardiogramm, das anschließend zur Rhythmusdetektion eingesetzt wird und gleichzeitig die Information der Elektrodenintegrität beinhaltet.

Die hier dargestellten Stromimpulse werden bereits für die intrakardiale Impedanzmessung z.B. für CLS eingesetzt und sind als klinisch unbedenklich einzustufen.

Die prinzipielle Möglichkeit, eine Kontraktilitätsgröße mittels intrakardialer Impedanzmessung zu erfassen ist u. a. in US 7,519,422 beschrieben.

## Patentansprüche

1. Implantierbares elektronisches Therapiegerät mit
- einer Therapieeinheit (460),
- einer Herzratenerfassungseinheit (450),
- einer Kontraktilitätsbestimmungseinheit (470) und
- einer Auswerte- und Steuereinheit (480),
von denen
die Therapieeinheit (460) ausgebildet ist eine antitachykarde Therapie zu liefern,
die Herzratenerfassungseinheit (440) dazu ausgebildet ist, aus einem Eingangssignal eine ventrikuläre Herzrate zu bestimmen
die Kontraktilitätsbestimmungseinheit (470) dazu ausgebildet ist, aus einem Eingangssignal ein Kontraktionssignal zu generieren, dass eine Kontraktilität einer Herzkammer widerspiegelt und
die Auswerte- und Steuereinheit (480) mit der Therapieeinheit (460), der Herzratenerfassungseinheit (450) und der Kontraktilitätsbestimmungseinheit (470) verbunden und ausgebildet ist, die Therapieeinheit (460) zur Abgabe einer antitachykarden Therapie anzusteuern, wenn
die Herzratenerfassungseinheit (450) einen Anstieg der Herzrate über einen vorbestimmten Schwellwertes erfasst und
die Kontraktilitätsbestimmungseinheit (470) ein Kontraktionssignal liefert, dass dem Anstieg der Herzrate nicht physiologisch adäquat ist.

2. Implantierbares elektronisches Therapiegerät nach Anspruch 1, **dadurch gekennzeichnet, dass** die Auswerte- und Steuereinheit (480) ausgebildet ist, eine Abgabe einer antitachykarden Therapie durch die Therapieeinheit (460) zu unterbinden, wenn die Herzratenerfassungseinheit (450) einen Anstieg der Herzrate über einen vorbestimmten Schwellwertes erfasst und die Kontraktilitätsbestimmungseinheit (470) ein Kontraktionssignal liefert, das anzeigt, dass die entsprechende Kontraktilität der Herzkammer gleichzeitig mit der Herzrate ansteigt.

3. Implantierbares elektronisches Therapiegerät nach Anspruch 1 oder 2, **dadurch gekennzeichnet, dass** das Kontraktionssignal eine Kontraktionsgeschwindigkeit einer Herzkammer repräsentiert.

4. Implantierbares elektronisches Therapiegerät nach einem der Ansprüche 1 bis 3, **dadurch gekennzeichnet, dass** die Auswerte- und Steuereinheit (480) ausgebildet ist, aus dem Kontraktionssignal einen Kontraktionsindex abzuleiten, der ein Verhältnis von einem Anstieg einer Herzrate zu einem Anstieg der Kontraktilität widerspiegelt.

5. Implantierbares elektronisches Therapiegerät nach Anspruch 4, **dadurch gekennzeichnet, dass** die Auswerte- und Steuereinheit (480) ausgebildet ist, den Kontraktilitätsindex in Kombination mit mindestens einem weiteren rhythmologischen oder IEGM-morphologischem Kriterium auszuwerten.

6. Implantierbares elektronisches Therapiegerät nach einem der Ansprüche 1 bis 5, **dadurch gekennzeichnet, dass** die Auswerte- und Steuereinheit (480) eine Rhythmusbewertungseinheit (450) aufweist, die ausgebildet ist einen Anstieg einer Herzrate über einen vorbestimmten Schwellwert zu erfassen.

7. Implantierbares elektronisches Therapiegerät nach einem der Ansprüche 1 bis 6, **dadurch gekennzeichnet, dass** die Kontraktilitätsbestimmungseinheit ausgebildet ist, das Kontraktionssignal mittels einer kontinuierlichen intrakardialen Impedanzmessung an einer ventrikulären Elektrode zu bestimmen.

8. Implantierbares elektronisches Therapiegerät nach einem der Ansprüche 1 bis 6, **dadurch gekennzeichnet, dass** die Kontraktilitätsbestimmungseinheit ausgebildet ist, das Kontraktionssignal mittels transkardialer Impedanzmessung zu bestimmen.

9. Implantierbares elektronisches Therapiegerät nach einem der Ansprüche 1 bis 6, **dadurch gekennzeichnet, dass** die Kontraktilitätsbestimmungseinheit ausgebildet ist, das Kontraktionssignal mittels transthorakaler Impedanzelektrokardiographie zu bestimmen.

10. Implantierbares elektronisches Therapiegerät nach einem der Ansprüche 1 bis 6, **dadurch gekennzeichnet, dass** die Kontraktilitätsbestimmungseinheit (470) ausgebildet ist, das Kontraktionssignal mittels intrakardialer Druckmessung zu bestimmen.

11. Implantierbares elektronisches Therapiegerät nach einem der Ansprüche 1 bis 10, **dadurch gekennzeichnet, dass** die Auswerte- und Steuereinheit (480) ausgebildet ist, eine Abgabe einer antitachykarden Therapie durch die Therapieeinheit (460) zu unterbinden, wenn Vergleich des intrakardialen Impedanzverlaufs bei Ruhe mit einem jeweils aktuellen intrakardialen Impedanzverlauf einen Herzratenanstieg indiziert, der unter Berücksichtigung einer vorgegebenen Toleranz einem aktuellen Herzratenanstieg entspricht.

12. Implantierbares elektronisches Therapiegerät nach einem der Ansprüche 1 bis 11, **dadurch gekennzeichnet, dass** das implantierbare elektronische Therapiegerät ein implantierbarer Cardioverter/Defibrillator, ein biventrikulärer Herzstimulator für die Resynchronisationstherapie und/oder ein antitachykarder Herzschrittmacher ist.

13. Implantierbares elektronisches Therapiegerät nach einem der Ansprüche 1 bis 12, **dadurch gekennzeichnet, dass** das implantierbare elektronische Therapiegerät eine Medikamentenpumpe aufweist.

14. Implantierbares elektronisches Therapiegerät nach einem der Ansprüche 1 bis 13, **dadurch gekennzeichnet, dass** das Implantierbare elektronische Therapiegerät ein Stimulator zur Modulation der kardialen Kontraktilität ist.

## Claims

1. An implantable electronic therapy device, comprising
- a therapy unit (460),
- a heart rate capturing unit (450),
- a contractility determination unit (470), and
- an evaluation and control unit (480),
wherein
the therapy unit (460) is designed to deliver an antitachycardiac therapy,
the heart rate capturing unit (440) is designed to determine a ventricular heart rate from an input signal,
the contractility determination unit (470) is designed to generate, from an input signal, a contraction signal which reflects a contractility of a ventricle, and
the evaluation and control unit (480) is connected to the therapy unit (460), the heart rate capturing unit (450), and the contractility determination unit (470) and designed to actuate the therapy unit (460) to administer an antitachycardiac therapy when the heart rate capturing unit (450) detects an increase in the heart rate above a specified threshold value and
the contractility determination unit (470) supplies a contraction signal which is not physiologically adequate for the increase in the heart rate.

2. An implantable electronic therapy device according to claim 1, **characterized in that** the evaluation and control unit (480) is designed to suppress an administration of an antitachycardiac therapy by the therapy unit (460) when the heart rate capturing unit (450) detects an increase in the heart rate above a specified threshold value and the contractility determination unit (470) supplies a contraction signal which indicates that the corresponding contractility of the ventricle rises together with the heart rate.

3. The implantable electronic therapy device according to claim 1 or 2, **characterized in that** the contraction signal represents a contraction rate of a ventricle.

4. An implantable electronic therapy device according to any one of claims 1 to 3, **characterized in that** the evaluation and control unit (480) is designed to derive a contraction index from the contraction signal that reflects a ratio of the increase in the heart rate to an increase in the contractility.

5. The implantable electronic therapy device according to claim 4, **characterized in that** the evaluation and control unit (480) is designed to evaluate the contractility index in combination with at least one further rhythmological or IEGM morphological criterion.

6. An implantable electronic therapy device according to any one of claims 1 to 5, **characterized in that** the evaluation and control unit (480) comprises a rhythm evaluation unit (450), which is designed to capture an increase in a heart rate above a specified threshold value.

7. An implantable electronic therapy device according to any one of claims 1 to 6, **characterized in that** the contractility determination unit is designed to determine the contraction signal by means of continuous intracardiac impedance measurement at a ventricular electrode.

8. An implantable electronic therapy device according to any one of claims 1 to 6, **characterized in that** the contractility determination unit is designed to determine the contraction signal by means of transcardiac impedance measurement.

9. An implantable electronic therapy device according to any one of claims 1 to 6, **characterized in that** the contractility determination unit is designed to determine the contraction signal by means of transthoracic impedance electrocardiography.

10. An implantable electronic therapy device according to any one of claims 1 to 6, **characterized in that** the contractility determination unit (470) is designed to determine the contraction signal by means of intracardiac pressure measurement.

11. An implantable electronic therapy device according to any one of claims 1 to 10, **characterized in that** the evaluation and control unit (480) is designed to suppress an administration of an antitachycardiac therapy by a therapy unit (460) when the comparison of the intracardiac impedance curve at rest to a current intracardiac impedance curve indicates an increase in the heart rate that corresponds to a current increase in heart rate, when taking a specified tolerance into consideration.

12. An implantable electronic therapy device according to any one of claims 1 to 11, **characterized in that** the implantable electronic therapy device is an implantable cardioverter/defibrillator, a biventricular cardiac stimulator for resynchronization therapy, and/or an antitachycardiac pacemaker.

13. An implantable electronic therapy device according to any one of claims 1 to 12, **characterized in that** the implantable electronic therapy device comprises a drug pump.

14. An implantable electronic therapy device according to any one of claims 1 to 13, **characterized in that** the implantable electronic therapy device is a stimulator for modulating the cardiac contractility.

## Revendications

1. Appareil thérapeutique électronique implantable, avec
- une unité thérapeutique (460),
- une unité de détection du rythme cardiaque (450),
- une unité de détermination de la contractilité (470), et
- une unité d'évaluation et de commande (480),
parmi lesquelles
l'unité thérapeutique (460) est conçue pour délivrer une thérapie antitachycardique, l'unité de détection du rythme cardiaque (440) est conçue pour déterminer un rythme cardiaque ventriculaire à partir d'un signal d'entrée, l'unité de détermination de la contractilité (470) est conçue pour générer un signal de contraction reflétant une contractilité ventriculaire, à partir d'un signal d'entrée, et
l'unité d'évaluation et de commande (480) est reliée à l'unité thérapeutique (460), à l'unité de détection du rythme cardiaque (450) et à l'unité de détermination de la contractilité (470), et conçue pour inciter l'unité thérapeutique (460) à délivrer une thérapie antitachycardique, lorsque l'unité de détection du rythme cardiaque (450) détecte une augmentation du rythme cardiaque au-delà d'une valeur seuil prédéterminée, et
l'unité de détermination de la contractilité (470) délivre un signal de contraction physiologiquement inadéquat pour l'augmentation du rythme cardiaque.

2. Appareil thérapeutique électronique implantable selon la revendication 1, **caractérisé en ce que** l'unité d'évaluation et de commande (480) est conçue pour empêcher une délivrance d'une thérapie antitachycardique par l'unité thérapeutique (460) lorsque l'unité de détection du rythme cardiaque (450) détecte une augmentation du rythme cardiaque au-delà d'une valeur seuil prédéterminée, et l'unité de détermination de la contractilité (470) délivre un signal de contraction indiquant que la contractilité correspondante du ventricule augmente avec le rythme cardiaque.

3. Appareil thérapeutique électronique implantable selon la revendication 1 ou 2, **caractérisé en ce que** le signal de contraction représente une vitesse de contraction d'un ventricule.

4. Appareil thérapeutique électronique implantable selon l'une des revendications 1 à 3, **caractérisé en ce que** l'unité d'évaluation et de commande (480) est conçue pour dériver un indice de contraction reflétant un rapport d'une augmentation d'un rythme cardiaque sur une augmentation de la contractilité, à partir du signal de contraction.

5. Appareil thérapeutique électronique implantable selon la revendication 4, **caractérisé en ce que** l'unité d'évaluation et de commande (480) est conçue pour évaluer l'indice de contractilité en combinaison avec au moins un autre critère ryhtmologique ou morphologique IEGM.

6. Appareil thérapeutique électronique implantable selon l'une des revendications 1 à 5, **caractérisé en ce que** l'unité d'évaluation et de commande (480) comporte une unité d'évaluation de rythme conçue pour détecter une augmentation du rythme cardiaque au-delà d'une valeur seuil prédéterminée.

7. Appareil thérapeutique électronique implantable selon la revendication 1 à 6, **caractérisé en ce que** l'unité de détermination de la contractilité est conçue pour déterminer le signal de contraction au moyen d'une mesure d'impédance intracardiaque continue sur une électrode ventriculaire.

8. Appareil thérapeutique électronique implantable selon l'une des revendications 1 à 6, **caractérisé en ce que** l'unité de détermination de la contractilité est conçue pour déterminer le signal de contraction au moyen d'une mesure d'impédance transcardiaque.

9. Appareil thérapeutique électronique implantable selon l'une des revendications 1 à 6, **caractérisé en ce que** l'unité de détermination de la contractilité est conçue pour déterminer le signal de contraction au moyen d'une électrocardiographie d'impédance transthoracique.

10. Appareil thérapeutique électronique implantable selon l'une des revendications 1 à 6, **caractérisé en ce que** l'unité de détermination de la contractilité (470) est conçue pour déterminer le signal de contraction au moyen d'une mesure de pression intracardiaque.

11. Appareil thérapeutique électronique implantable selon l'une des revendications 1 à 10, **caractérisé en ce que** l'unité d'évaluation et de commande (480) est conçue pour empêcher une délivrance d'une thérapie antitachycardique par l'unité thérapeutique (460) lorsque la comparaison entre l'évolution de l'impédance intracardiaque au repos et l'évolution de l'impédance intracardiaque respectivement actuelle indique une augmentation du rythme cardiaque correspondant à une augmentation actuelle du rythme cardiaque en tenant compte d'une tolérance prédéfinie.

12. Appareil thérapeutique électronique implantable selon l'une des revendications 1 à 11, **caractérisé en ce que** l'appareil thérapeutique électronique implantable est un cardioverteur/défibrillateur implantable, un stimulateur cardiaque biventriculaire pour la thérapie de resynchronisation et/ou un stimulateur cardiaque antitachycardique.

13. Appareil thérapeutique électronique implantable selon l'une des revendications 1 à 12, **caractérisé en ce que** l'appareil thérapeutique électronique implantable comporte une pompe à médicaments.

14. Appareil thérapeutique électronique implantable selon l'une des revendications 1 à 13, **caractérisé en ce que** l'appareil thérapeutique électronique implantable est un stimulateur pour la modulation de la contractilité cardiaque.
